# EUROPEAN PATENT SPECIFICATION

(11) **EP 3 377 075 B1**
(45) Date of publication and mention of the grant of the patent: **06.03.2024**
(21) Application number: 16816746.8
(22) Date of filing: 18.11.2016
(51) Int. Cl.: A61K 31/711, A61P 29/00

(54) **COMPOSITION FOR THE TREATMENT OF INFLAMMATORY BOWEL DISEASE**
ZUSAMMENSETZUNG ZUR BEHANDLUNG VON ENTZÜNDLICHER DARMERKRANKUNG
COMPOSITION POUR LE TRAITEMENT DES MALADIES INFLAMMATOIRES DE L'INTESTIN

(30) Priority: 20.11.2015 IT UB20155754
(43) Date of publication of application: 26.09.2018
(73) Proprietor: Mastelli S.r.l., 18038 Sanremo (IM) (IT)
(72) Inventor: SQUADRITO, Francesco, 98122 Messina (IT); CATTARINI MASTELLI, Giulia, 16122 Genova (IT); CATTARINI MASTELLI, Laura, 18038 Sanremo (Imperia) (IT); ALTAVILLA, Domenica, 98122 Messina (IT); BITTO, Alessandra, 98123 Messina (IT)
(74) Representative: Rambelli, Paolo
(86) International application number: PCT/IB2016/056942
(87) International publication number: WO 2017/085670

(56) References cited:
- EP-A2- 0 226 254
- WO-A1-99/22741
- WO-A2-01/79487
- E RIJCKEN: "ICAM-1 and VCAM-1 antisense oligonucleotides attenuate in vivo leucocyte adherence and inflammation in rat inflammatory bowel disease", GUT, vol. 51, no. 4, 1 October 2002 (2002-10-01), pages 529-535, XP055075942, ISSN: 0017-5749, DOI: 10.1136/gut.51.4.529
- DATABASE BIOSIS [Online] BIOSCIENCES INFORMATION SERVICE, PHILADELPHIA, PA, US; May 2011 (2011-05), TAKEDATSU HIDETOSHI ET AL: "The New Therapeutic Approach for Inflammatory Bowel Disease Using Antisense Macrophage-Migration Inhibitory Factor (MIF)/Schizophyllan (SPG) Complex", XP002760243, Database accession no. PREV201100404086 & GASTROENTEROLOGY, vol. 140, no. 5, Suppl. 1, May 2011 (2011-05), page S520, DIGESTIVE DISEASE WEEK 2011; CHICAGO, IL, USA; MAY 07 -10, 2011 ISSN: 0016-5085
- BELO A ET AL: "GASTROPROTECTIVE EFFECTS OF ORAL NUCLEOTIDE ADMINISTRATION", GUT, BRITISH MEDICAL ASSOCIATION, LONDON, UK, vol. 55, no. 2, 1 February 2006 (2006-02-01), pages 165-171, XP009064654, ISSN: 0017-5749, DOI: 10.1136/GUT.2005.076752
- ALESSANDRA BITTO ET AL: "Polydeoxyribonucleotide reduces cytokine production and the severity of collagen-induced arthritis by stimulation of adenosine A2A receptor", ARTHRITIS & RHEUMATISM, vol. 63, no. 11, 28 October 2011 (2011-10-28), pages 3364-3371, XP055290893, US ISSN: 0004-3591, DOI: 10.1002/art.30538
- LAZZAROTTO M ET AL: "Clinical evaluation of corneal epithelialization after photorefractive keratectomy in patients treated with polydeoxyribonucleotide (PDRN) eye drops: A randomized, double-blind, placebo-controlled trial", EUROPEAN JOURNAL OF OPHTHALMOLOGY, WICHTIG PUBLISHING, IT, vol. 14, no. 4, 1 July 2004 (2004-07-01), pages 284-289, XP008134970, ISSN: 1120-6721
- GUIZZARDI STEFANO ET AL: "Polydeoxyribonucleotide (PDRN) promotes human osteoblast proliferation: A new proposal for bone tissue repair", LIFE SCIENCES, PERGAMON PRESS, OXFORD, GB, vol. 73, no. 15, 29 August 2003 (2003-08-29), pages 1973-1983, XP002583525, ISSN: 0024-3205

## Description

The present invention relates to a new therapeutic use of polydeoxyribonucleotides (PDRNs) for the treatment of inflammatory bowel disease, particularly Crohn's disease and ulcerative colitis.

Inflammatory Bowel Disease (IBD) such as Crohn's disease and ulcerative colitis is a group of chronic syndromes of the gastrointestinal tract, characterized by inflammation of the enteric wall, abdominal pain, diarrhoea, bleeding and malabsorption. The aetiology of these diseases is not yet known, even if there is increasing evidence that IBD originates from altered immunological, genetic and environmental factors.

A growing body of data indicates that oxygen- and nitrogen-derived free radicals, such as superoxide, hydroxyl radicals and nitric oxide, are involved in the pathogenesis of IBD. The pro-inflammatory role of these reactive species is well known, including the recruitment of neutrophils at the site of inflammation, lipid peroxidation and the release of chemotactic factors and cytokines. During inflammation, ROS represent an important signal for the activation of NF-kB, which in turn causes adaptive modifications of the damaged cells, such as expression of genes for proinflammatory mediators and programmed cell death.

Conventional therapies for IBD include anti-inflammatory drugs, such as aminosalicylates, corticosteroids, thiopurines, methotrexate and anti-tumour necrosis factor agents. The long-term use of the above-mentioned drugs, however, can cause serious side effects, with a negative impact on the quality of life. For this reason, there is a need to seek new effective approaches with fewer side effects.

Earlier studies have shown that stimulation of the adenosine receptor is important in decreasing inflammation. The different receptor subtypes (A₁, A_{2A}, A_{2B}, A₃) are expressed on immune/inflammatory cells, such as lymphocytes, neutrophils, monocytes and macrophages. It was also demonstrated that A_{2A} is the main receptor responsible for the inhibitory effect of adenosine on the production of proinflammatory cytokines in different experimental disease models, including asthma, ischaemia, arthritis and sepsis. All these findings encourage the search for new drugs suitable for the treatment of IBD, through pharmacological activation of A_{2A} receptors.

E. Rijcken, GUT, vol. 51, No. 4, 1 October 2002, PAGES 529-535, discloses the use of antisense oligonucleotides against ICAM-1 and VICAM-1 as potential candidates for the treatment of inflammatory bowel diseases; these have generally a chain length from 15 to 25 bases.

Hidetoshi et al., BIOSCIENCE INFORMATION SERVICE, Philadelphia, PA, May 2011, discloses the possibility of using the antisense MIF/SPG complex in the treatment of inflammatory bowel disease. Antisense MIF/SPG is a complex formed by the antisense oligonucleotide against the macrophage-migration inhibitory factory (MIF) with the schizophyllan (SPG) polysaccharide.

WO99/22741 discloses the use of a deoxyribonucleoside, deoxyribonucleotide (DNA monomer) or oligodeoxyribonucleotide (short length DNA) to improve DNA repair of skin or mucosa.

WO01/79487 relates to the use of polydeoxyribonucleotides as oligonucleotides which build a triple helix with at least a nucleic acid sequence specific for the transcription region or promotor region of the ICAM-1 gene, where the polydeoxyribonucleotide has at least three consecutive purinic or pyrinidinic bases, in the treatment of ICAM-associated disease, among which the Crohn disease; said oligonucleotides comprise from 10 to 35 nucleotides.

Belo et al., GUT, BRITISH MEDICAL ASSOCIATION, London, UK, vol. 55, No. 2, 1 February 2008, pages 165-171) suggests the potential use of nucleotides (NT) for the treatment of ulcerative conditions of the bowel.

The object of the present invention is to provide a pharmaceutical composition for use in the therapeutic treatment of inflammatory bowel disease, which is substantially free of side effects, even in the long term.

Therefore, the invention is based on the finding that the administration of polynucleotides, particularly polydeoxyribonucleotides (PDRNs) in an experimental colitis animal model (in rats) causes a significant improvement in all pathological outcomes associated with inflammatory colitis.

Therefore, an object of the invention is a composition of polynucleotides for use in the therapeutic treatment of inflammatory bowel disease in an individual suffering from this disease, as defined in the claims that follow.

The term polynucleotides, and particularly polydeoxyribonucleotides, is intended to mean a composition or mixture of polymer chains with chain lengths generally of from 50 to 2000 base pairs, representing a source of purine, pyrimidine, nucleosides and nucleotides.

Said composition comprises polydeoxyribonucleotides with different molecular weights and has a molecular weight distribution of the deoxyribonucleotide polymer chains of from 0.5 kDaltons to 3000 kDaltons, more preferably from 30 kDaltons to 2000 kDaltons, with a number average molecular weight of from 250 to 400 kDaltons, more preferably of approximately 350 kDaltons. Said DNA polymer chain moiety is obtained by extraction from fish sperm or plants, by means of a process which comprises a step of partial fragmentation of the DNA chains, in order to reduce the molecular weights thereof, and a step of, at least partial, depurination of the DNA, to remove the informational capacity thereof.

The preferred percentage of DNA depurination is preferably of from 1% to 5% of purine bases removed (i.e. apurinic sites), based on the total of the purine bases originally present. A depurination percentage of between 2 and 3% being preferred.

Therefore, the PDRN mixture used is devoid of informational capacity as a result of the depurination and is subjected to sterilization techniques for viruses and embryos and to a purification method that preferably results in a dry purity higher than 95%, typically of approximately 98%, with protein amounts of less than 0.5%.

Polynucleotide or PDRN compositions for use by intramuscular or subcutaneous administration are commercially available under the trade names Placentex@ (intact) and Placentex^{®} 50%. This drug has a toxicity level not detectable at the highest administrable dose; acute toxicity studies in rats and mice failed to identify the LD50, which was higher than the highest administrable dose (30 ml/kg Placentex@ intact). Chronic toxicity studies by the systemic route based on 1 ml/kg/day of the same preparation showed no deaths or evident diseases in the individual macro- and microscopically studied organs, or even general health disorders.

The activity of the polynucleotide composition in the therapeutic use according to the invention was assessed by means of the following experimental tests carried out in experimental colitis models.

In the accompanying drawings:
- Figure 1: colon tissues from the sham group (A), DNBS vehicle (B), DNBS+drug vehicle (C), DNBS+PDRN (D), DNBS+DMPX+PDRN (E), together with the chart that represents the macroscopic damage scores (F). The values obtained from seven animals per group are expressed as the mean and SEM.*p<0.0001 vs DNBS+drug vehicle group, #p<0.0001 vs sham group;
- Figure 2: food intake and body weight assessed during the experimental period.

The values were obtained from seven animals per group and are expressed as the mean and SD. Food intake in DNBS animals (A) *p<0.0001 vs DNBS+drug vehicle group p<0.0001 vs sham group.

Weight loss in DNBS animals (B) §p<0.05 vs DNBS+drug vehicle, *p<0.0001 vs DNBS+drug vehicle group, *p<0.0001 vs sham group.

Food intake in DSS animals (C) **p<0.0001 vs DSS+drug vehicle group, °p<0.0001 vs sham group, ^p<0.0001 vs sham group.

Weight loss in DSS animals (D) §§p<0.05 vs DSS+drug vehicle group, **p<0.0001 vs DSS+drug vehicle group, °p<0.001 vs sham group, ^p<0.0001 vs sham group;

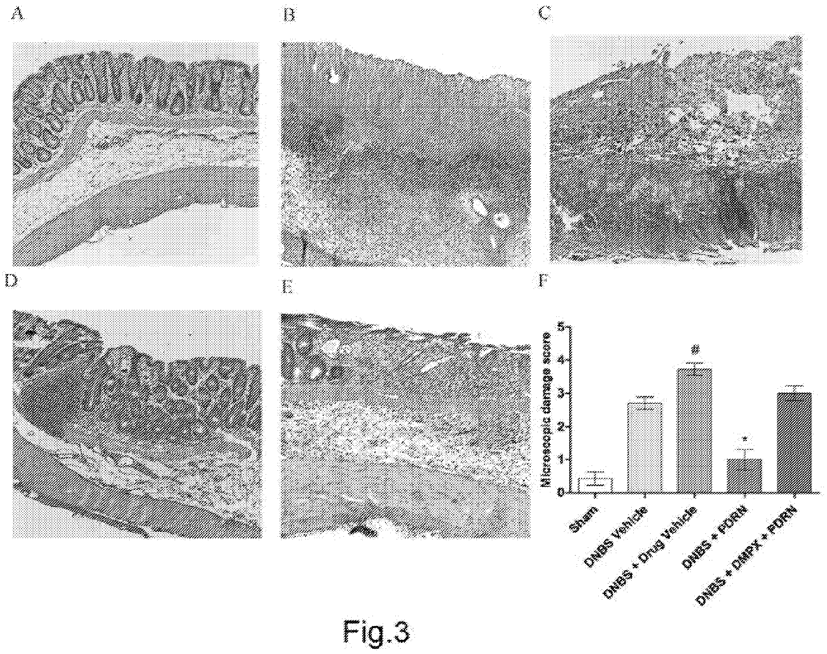

- Figure 3: histological evaluations (H&E staining, original magnification x10) from the sham group (A), DNBS vehicle (B), DNBS+drug vehicle (C), DNBS+PDRN (D), DNBS+DMPX+PDRN (E). The charts represent the microscopic damage score (F), the values are expressed as the mean and SEM of seven animals. *p<0.0001 vs DNBS+drug vehicle group, #p<0.0001 vs sham group;

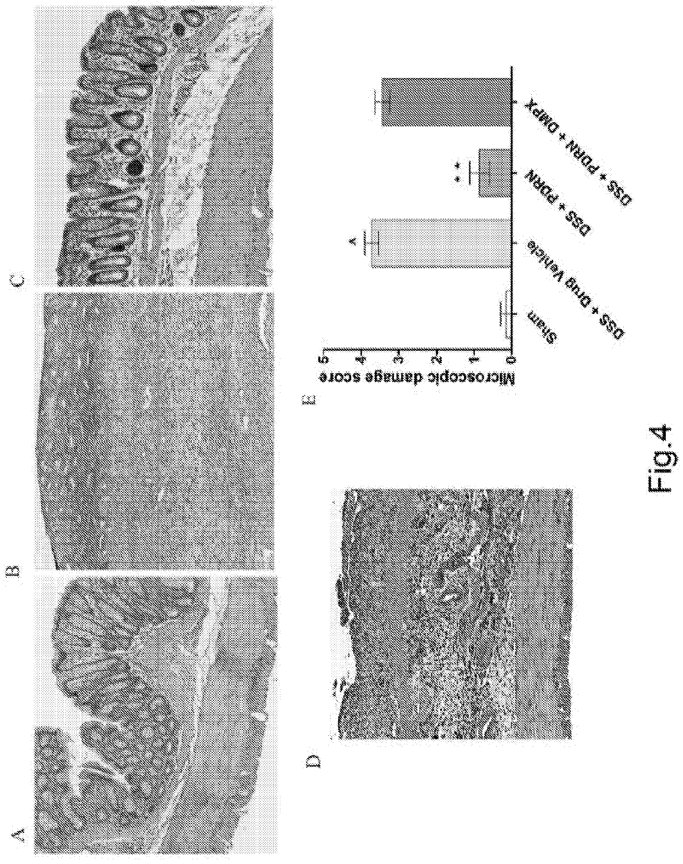

- Figure 4: histological evaluations (H&E staining, original magnification x10) from the sham group (A), DSS+drug vehicle (B), DSS+PDRN (C), DSS+DMTX+PDRN (D). The chart represents the microscopic damage score (E), the values are expressed as the mean and SEM of seven animals. **p<0.0001 vs DSS+drug vehicle group, ^p<0.0001 vs sham group;

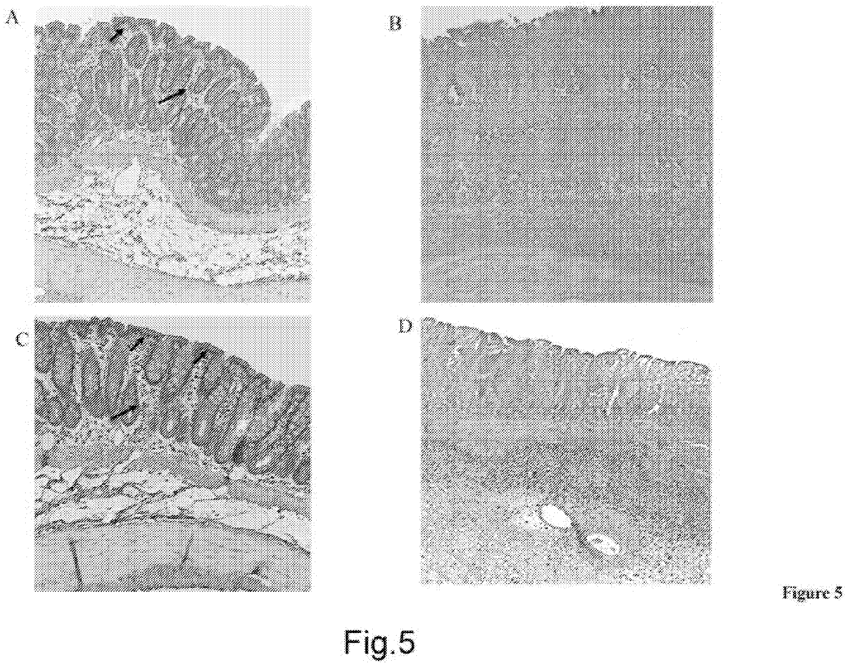

- Figure 5: immunohistochemical evaluation of Bax (magnification x10) from the sham group (A), DNBS vehicle (B), DNBS+PDRN (C), DNBS+DMPX+PDRN (D). The arrows at the points A and C point out the slight positivity. The figures in B and D show a diffuse staining of the apoptotic factor;

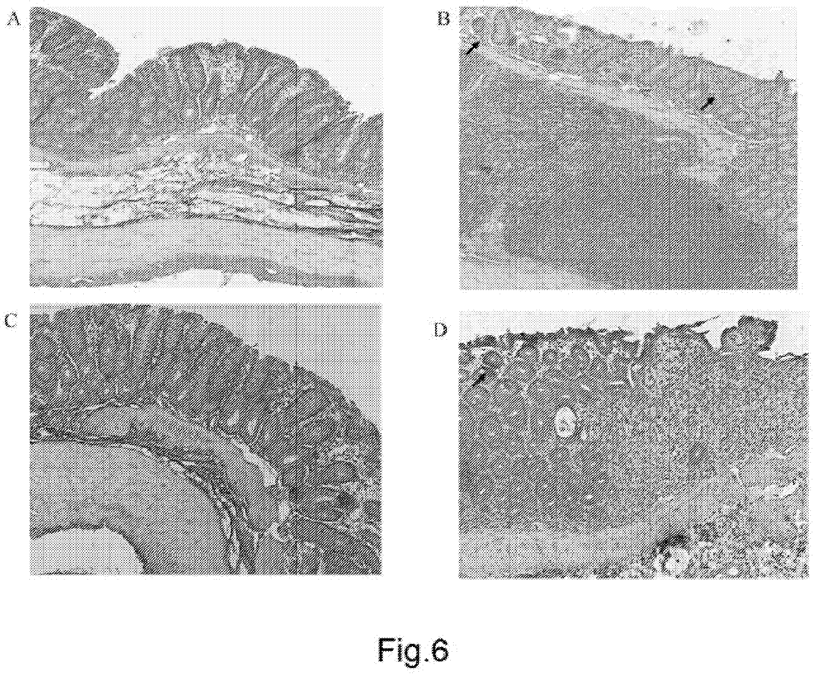

- Figure 6: immunohistochemical evaluations of Bcl-2 (magnification x10) from the sham group (A), DNBS vehicle (B), DNBS+PDRN (C), DNBS+DMPX+PDRN (D). The image in A and C shows a diffuse staining for Bcl-2, while the arrows in B and D point to the residual staining of Bcl-2 in the mucosal layer;

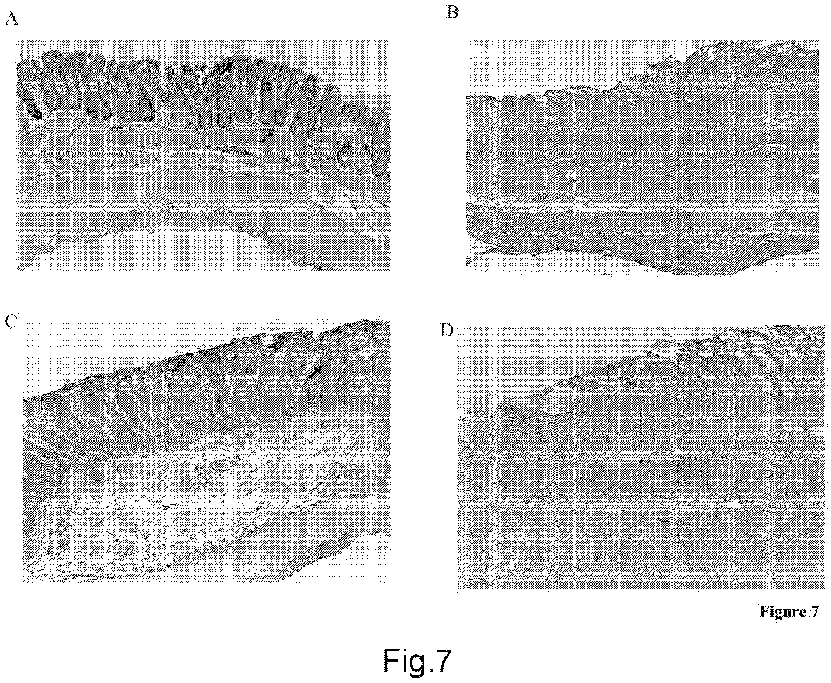

- Figure 7: immunohistochemical evaluation of Bax (magnification x10) from the sham group (A), DSS+drug vehicle (B), DSS+PDRN (C), DSS+DMPX+PDRN (D). The arrows in A and C point out the slight positivity. Figures B and D show a diffuse staining of the apoptotic factor;

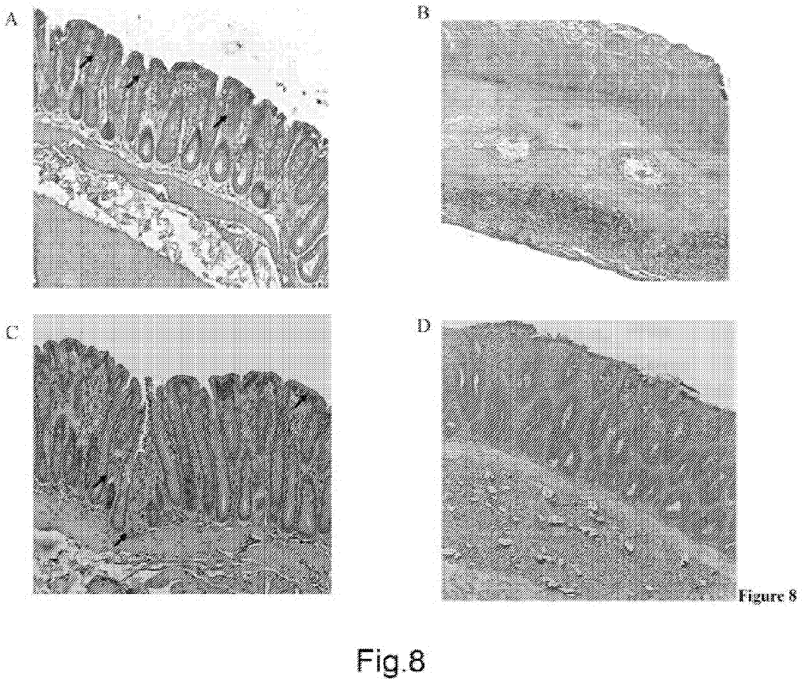

- Figure 8: immunohistochemical evaluations of Bcl-2 (magnification x10) from the sham group (A), DSS+drug vehicle (B), DSS+PDRN (C), DSS+DMPX+PDRN (D). The image in A and C shows a diffuse staining for Bcl-2, while the arrows in B and D point to the residual staining of Bcl-2 in the mucosal layer;

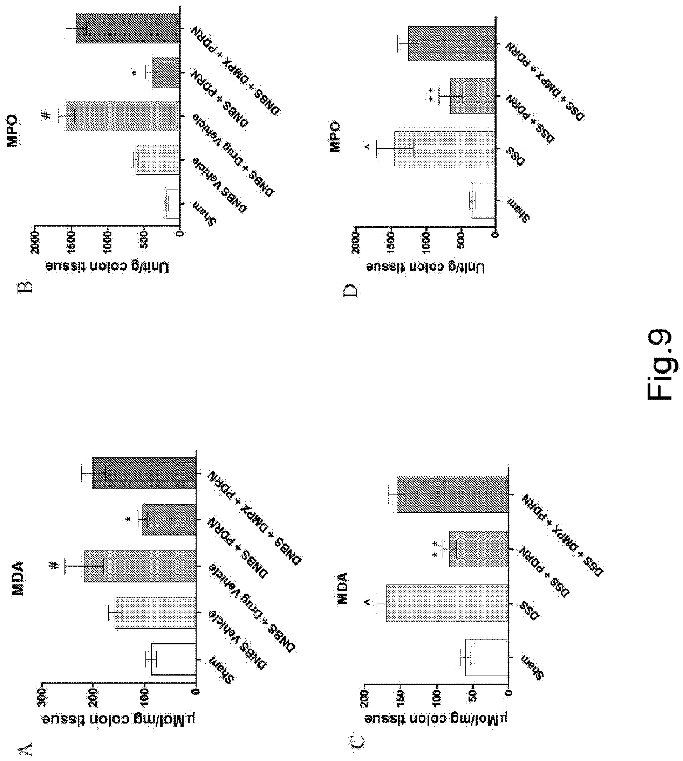

- Figure 9: effects of PDRNs on malondialdehyde levels (A) and myeloperoxidase activity (B) in DNBS animals. *p<0.0001 vs DNBS+drug vehicle group, #p<0.0001 vs sham group. Effects of PDRNs on malondialdehyde levels (C) and myeloperoxidase activity (D) in DSS animals. **P<0.0001 vs DSS+drug vehicle group, *p<0.0001 vs sham group. The values are expressed as the mean and SD of seven animals;

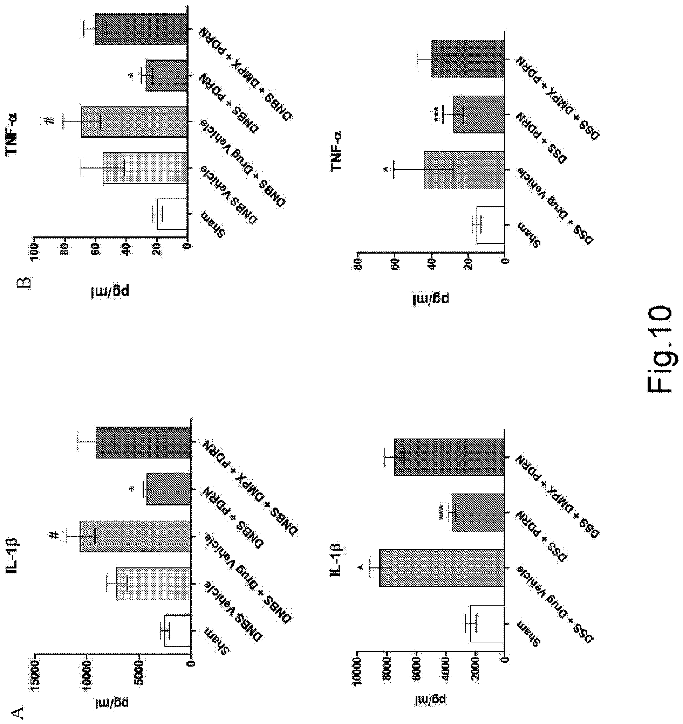

- Figure 10: IL-1 β (A and C) and TNF-α (B and D) levels in the serum of DNBS- and DSS-treated animals. The values are expressed as the mean and SD of seven animals. #p<0.0001 vs sham group, *p<0.0001 vs DNBS+drug vehicle group. ^p<0.0001 vs sham group, ***p<0.01 vs DSS+drug vehicle group.

### Experimental tests

### Animals and treatments

All animal procedures were performed in accordance with the Principles of Laboratory Animal Care (NIH Publication No. 85/23, revised 1985) and were in agreement with the ARRIVE guidelines.

A total of 63 male Sprague Dawley rats (250-300 g) were purchased from Charles River Laboratories (Calco, Milan, Italy). The animals were kept in plastic cages under standard environmental conditions and fed *ad libitum* at the animal facility of the University of Messina. The animals were allowed to acclimate for 1 week prior to the beginning of the experiments.

DNBS colitis was induced in fasted rats under mild anaesthesia by means of a single intra-colon instillation of 2,4-dinitrobenzenesulfonic acid (DBNS; Sigma-Aldrich, 25 mg, 0.8 ml and 50% ethanol) via a catheter that was inserted into the colon through the anus up to the splenic flexure, 8 cm from the anus. The animals were then maintained for 15 minutes in the Trendelenburg position to avoid reflux and after 6 hours they were randomly divided so as to receive, by the i.p. route, the drug vehicle (1 ml/kg; n=7), PDRN (8 mg/kg; n=7), or PDRN (8 mg/kg; n=7) + 3,7-dimethyl-1-propargylxanthine (DMPX; 10 mg/kg; n=7), an A_{2A} receptor antagonist.

The animals of the sham group (n=7) or of the DNBS vehicle group (n=7) received a single intra-colon instillation of 0.8 ml saline solution or 50% ethanol, respectively. The animals were observed for colitis symptoms, such as diarrhoea and appetite, for 7 days and were then sacrificed and enteric tissue and blood samples were collected for analysis.

In a second series of experiments, colitis was induced in 21 rats by oral administration of 8% DSS (MP Biomedicals) in the drinking water from day 0 to day 5. The body weight, stool consistency and blood in the stool were monitored daily for determining the degree of colitis. After 24 hours, the animals were randomly divided as previously reported. The animals of the sham group (n=7) received standard drinking water *ad libitum.*

On the day of sacrifice, the abdomen was opened through an incision in the midline and the descending colon was removed, opened along the antimesenteric edge, rinsed and cut into two equal pieces, one for histological examination and the other for biochemical marker analyses and also blood samples were collected from the heart to assess proinflammatory cytokine levels.

The PDRN composition used was a composition corresponding to the commercial product Placentex@ (intact) from Mastelli S.r.l., freshly prepared, available in 3 ml vials containing 5.6 mg of PDRNs, in accordance with the molecular weight indication previously reported in an aqueous solution containing 0.9% sodium chloride. The drug was administered starting after 6 hours or 24 hours of treatment with DNBS or DSS, respectively. The doses and routes of administration were selected in agreement with what is stated in the publication by Bitto A, et al in Arthritis Reum, 2011; 63:3364-3371.

### Body weight and food intake assessments

The body weight and food intake were recorded daily between 9 a.m. and 10 a.m. from the day of colitis induction until the end of the experiment. The body weight results were expressed as raw data and compared with the food intake, by using the following formula: food intake divided by body weight in grams and multiplied by 100.

### Macroscopic damage score

The damage to the colon was assessed by two independent observers in accordance with the following criteria: 0 (no damage), 1 (localised hyperaemia without ulcers), 2 (linear ulcers with no significant inflammation), 3 (linear ulcers with inflammation at one site), 4 (two or more major sites of inflammation and ulceration extending for 1 cm along the length of the colon) and 5-8 (one point is added for each centimetre of ulceration in addition to the initial 2 cm).

### Microscopic damage score

For light microscopy, tissues were rapidly removed from the colon and fixed in 10% buffered formalin. Subsequently, the samples were embedded in paraffin, sectioned to a thickness of 5 µm and stained with haematoxylin and eosin and observed under a Leica microscope.

The evaluation of tissue changes was carried out by two observers blinded to the experimental protocol. The following morphological criteria were considered: score 0 (no damage), score 1 (mild focal necrotic epithelial oedema), score 2 (moderate diffuse swelling and necrosis of the villi), score 3 (severe necrosis with presence of neutrophil infiltration in the submucosa), score 4 (very severe diffuse necrosis with massive neutrophil infiltration and haemorrhage).

### Immunohistological evaluation of Bax and Bcl-2

The tissues embedded in paraffin were sectioned (5 µm), rehydrated, and antigen recovery was carried out using 0.05 M sodium citrate buffer (pH 6.0). The tissues were treated with 1% hydrogen peroxide, to block the activity of exogenous peroxidase, and with normal horse serum (Vector Laboratories), to prevent non-specific staining. A primary antibody against Bax and Bcl-2 (Abcam) (Cell Signaling Technologies) was used and kept overnight at 4°C in a wet container. After washing in PBS, a secondary antibody (Vector Laboratories) was used and the position of the reaction was visualised with diaminobenzidine tetrahydrochloride (Sigma-Aldrich). The cover slips were counterstained with hematoxylin, dehydrated and mounted on slides. As part of the histological evaluation, all the slides were examined by a pathologist having no knowledge of the previous treatment using masked slides with a magnification from 5x to 40x under a Leica microscope.

### Myeloperoxidase activity measurements

The activity of myeloperoxidase (MPO), an indicator of the accumulation of polymorphonuclear leukocytes, was determined as described in the literature (Mullane et al, J. Pharmacol Methods, 1985; 14:157-67). Equal amounts of colon tissue were homogenized in a solution containing 0.5% hexadecyltrimethylammonium bromide dissolved in 10mM potassium phosphate buffer (pH 7.0). The lysates were then centrifuged for 30 minutes at 15000 rpm at 4°C. An aliquot of the supernatant was reacted with a solution of 1.6 mM tetramethylbenzidine and 0.1 mM H₂O₂. The absorbance change rate was measured by spectrophotometry at 650 mM. MPO activity was defined as the amount of enzyme which degrades one µmole of hydrogen peroxide/minute at 37°C and was expressed as units per g of tissue.

### Malondialdehyde measurement

Malondialdehyde (MDA) levels in the colon were determined as an indicator of lipid peroxidation. Equal amounts of colon tissues were homogenized in 1.5% KCl solutions, a 0.1 ml aliquot of the homogenate was added to a reaction mixture containing 0.2 ml of 8.1% SDS, 1.5 ml of 20% acetic acid, 1.5 ml of 0.8% thiobarbituric acid and 700 ml of distilled water. The samples were boiled for 1 hour at 95°C and centrifuged at 3000 g for 10 minutes. The absorbance of the supernatant was measured by a spectrophotometer at 650 nm.

### IL-1β and TNF-α assessment in serum

The samples were tested by using a commercially available ELISA kit for IL-1β and TNF-α (Abeam). The results were obtained by interpolating the absorbances with standard curves and expressed in pg/ml in both cases.

### Statistical analysis

All quantitative data are expressed as the mean ± SD or the mean ± SEM for each group and compared by using a one-way or two-way ANOVA for non-parametric variables with Tukey's post-test for intergroup comparisons. The statistical significance was set at p<0.05. Charts were designed using the GraphPad Prism software, version 5.0 for Windows.

### Results

### PDRN effects on body weight, food intake and macroscopic damage

Colitis was successfully induced in rats following intra-colon treatment with DNBS, as demonstrated by the appearance of diarrhoea after only 6 hours and by a significant body weight loss at the end of the experiment (p<0.0001 vs sham group).

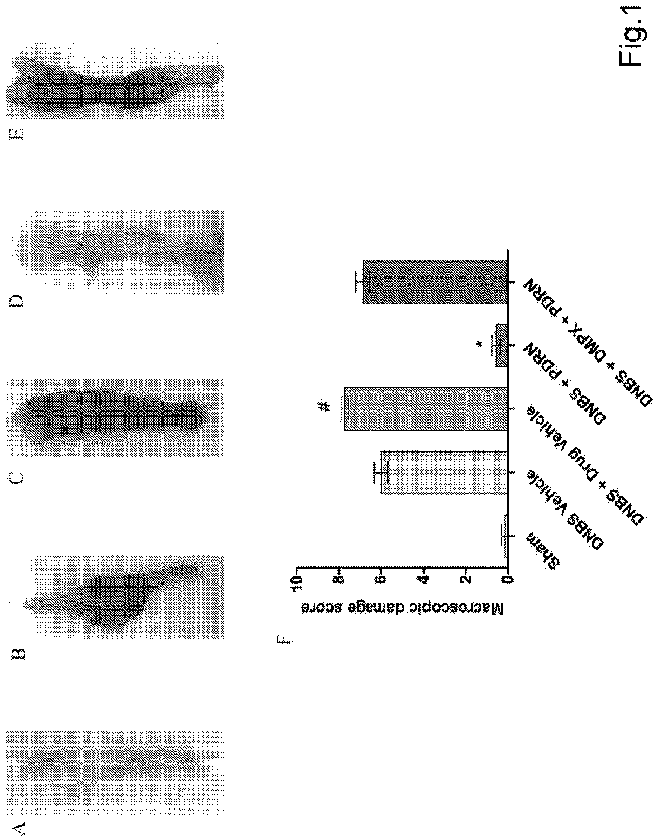

Treatment with PDRNs resulted in weight gain and appearance of solid stool after day 3 of treatment. 7 days after colitis induction, in a macroscopic observation, the colonic mucosa of animals in the DNBS plus drug vehicle group and in the DNBS group appeared ulcerated and oedematous, and hyperemic compared to the sham group (p<0.0001, Figures 1A-C). In the PDRN-treated group there was a significant reduction in the extent and seriousness of the damage to the colon (p<0.0001 vs the DNBS + drug vehicle group). In addition, the effect of PDRN administration on the macroscopic damage was attenuated by the A_{2A} receptor antagonist (Figures 1D-E-F).

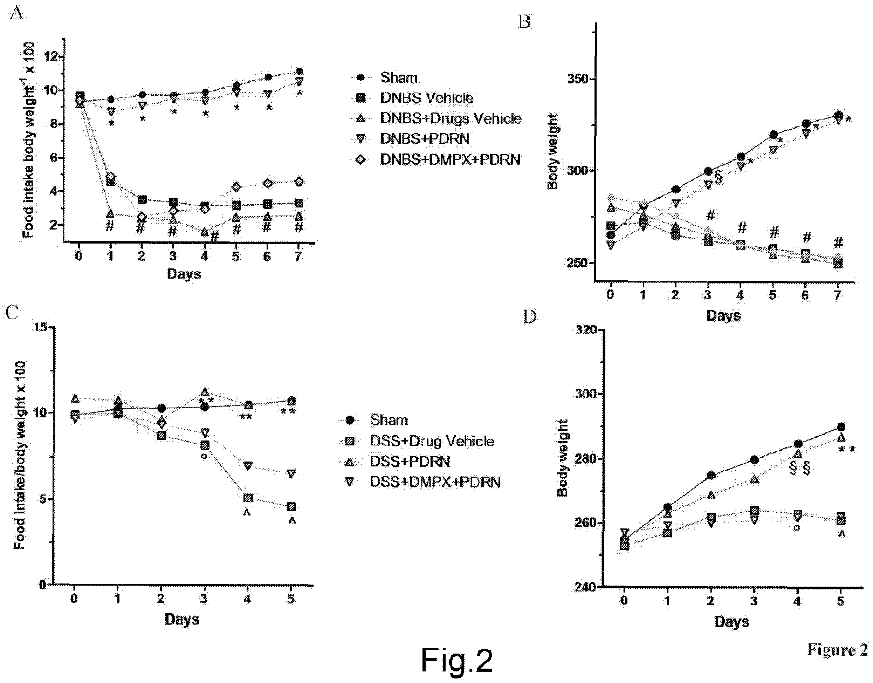

In the DNBS model, food intake and body weight were recorded at the baseline (Day 0) and daily throughout the experimentation period. Food intake is shown in Figure 2A and the body weight data are reported in Figure 2B. Treatment with PDRNs caused an increase in weight and food intake during the treatment period (p<0.0001 vs the DNBS+drug vehicle group at the end of the experiment; Figures 2A-B). DMPX abolished the positive effect of PDRNs on food intake and body weight.

Animals administered with DSS showed diarrhoea and rectal bleeding after 24 hours, while weight loss was observed starting from day 3 (p<0.0001 at the end of the experiment vs sham group). Treatment with PDRNs resulted in reduced bleeding and increased food intake and body weight (p<0.0001 vs the DSS+drug vehicle group at the end of the experiment). The positive effects of PDRNs were attenuated by concomitant administration of the A_{2A} antagonist, DMPX (Figures 2C and 2D).

### PDRNs reduce the histological damage

The sham group (Figure 3A) showed a normal appearance of the colon mucosa with intact epithelium. In the DNBS-vehicle group (Figure 3B), the histological analysis carried out 7 days after colitis induction showed damage to the mucosa with alterations in the structure of the enteric tissue and signs of inflammation. The damage to the mucosa induced by administration of DNBS was characterized by epithelium necrosis and massive infiltration of neutrophils and macrophages in the mucosa and submucosa layers, thickening of the colon wall, transmural necrosis, loss of goblet cells and oedema (Figure 3C). The administration of PDRNs significantly reduced the extent and seriousness of the histological alteration associated with the administration of DNBS, stimulating epithelium regeneration with preservation of villi, crypts and goblet cells (Figure 3D). The block of the A_{2A} receptors with DMPX countered the beneficial effect of PDRNs in animals with DNBS colitis (Figure 3E). The microscopic damage score of the DNBS+PDRN group was significantly reduced when compared with the DNBS+drug vehicle group (p<0.0001; Figure 3F).

In the DSS model, the histological analysis carried out after 5 days showed a flattening of the villi with reduced presence of mucosal glands, massive inflammatory infiltrate and oedema in the submucosal layer, with no obvious signs of apoptosis (Figure 4B), in comparison with sham animals (Figure 4A). Treatment with PDRNs significantly reduced the extent and seriousness of the histological alteration associated with the administration of DSS, stimulating regeneration of the epithelium of villi, crypts and goblet cells (Figure 4C). The administration of DMPX abolished the protective effects of the PDRNs on the histological scores (Figure 4D). The microscopic damage score of the DSS+PDRN group was significantly reduced when compared with the DSS+drug vehicle group (p<0.0001; Figure 4E).

### Effects of PDRNs on immunohistochemical evaluations of Bax and Bcl-2.

Colon samples were collected 7 days after administration of DNBS in order to determine the immunohistological staining for Bax and Bcl-2. Tissues obtained from sham rats showed a very low staining for Bax (Figure 5A), while colon sections obtained from the DNBS vehicle group (not shown) and from the DNBS+drug vehicle group exhibited a positive staining for Bax (Figure 5B). PDRNs markedly reduced the degree of Bax-positive staining in the colon of rats administered with DNBS (Figure 5C), while the co-administration of DMPX abolished the effect of PDRNs (Figure 5D). Moreover, the staining of Bcl-2 in the colon section from the sham group was highly positive (Figure 6A); while the DNBS vehicle group (not shown) and the DNBS+drug vehicle group were slightly positive for the anti-apoptotic factor (Figure 6B). Treatment with PDRNs restored the presence of Bcl-2 (Figure 6C). The effect of PDRNs was markedly contrasted by the concomitant administration of DMPX (Figure 6D). In the DSS model, the immune labelling, performed after 5 days, showed a slight staining for Bax in the sham group (Figure 7A). DSS induced expression of Bax (Figure 7B) which was reduced by treatment with PDRNs to basal levels (Figure 7C). The co-administration of DMPX attenuated the effects of PDRNs and the expression of Bax (Figure 7D). Sham animals were highly positive for Bcl-2 (Figure 8A), while a significant reduction was seen in the DSS group (Figure 8B). Animals treated with PDRNs showed a renewed positivity for Bcl-2 (Figure 8C) and this effect was abolished by the A_{2A} antagonist (Figure 8D).

### Effects of PDRNs on lipid peroxidation and neutrophil infiltration

MDA levels were increased as expected both in the DNBS-vehicle group and in the DNBS+drug vehicle group (p<0.0001 vs sham), while treatment with PDRNs lowered lipid peroxidation (p<0.0001 vs DNBS+drug vehicle). Also the accumulation of polymorphonuclear granulocytes, determined by the activity of myeloperoxidase, was increased in the DNBS vehicle group and in the DNBS+drug vehicle group (p0.0001 vs sham). Treatment with PDRNs significantly reduced the levels of MPO and MDA (p<0.0001 vs DNBS+drug vehicle), the co-administration of DMPX lowered this positive effect (Figures 9A-B).

MDA levels were increased (p<0.0001 vs sham) in the DSS+drug vehicle group. The administration of PDRNs decreased lipid peroxidation (p<0.0001). The accumulation of neutrophils, determined by MPO, was increased in the DSS+drug vehicle group (p<0.0001 vs sham). PDRNs significantly reduced the activity of MPO (p<0.0001 vs DSS+drug vehicle group). DMPX abolished the positive effects of PDRNs both on the MDA level and on the activity of MPO (Figures 9C-D).

### Effects of PDRNs on IL-1 β and TNF-α

The circulation levels of the pro-inflammatory cytokines IL-1 β and TNF-α were measured 7 days after administration of DNBS. As shown in Figures 10A and 10B, a significant increase of both IL-1 β and TNF-α was seen in the serum from both the DNBS-vehicle and DNBS plus drug vehicle groups in comparison with control animals (p<0.0001 vs sham). As a result of A_{2A} stimulation by PDRNs, the serum levels of IL-1 β and TNF-α were significantly reduced (p<0.0001 vs DNBS+drug vehicle group). PDRNs failed to elicit an inhibitory effect on the cytokine levels in the presence of DMPX (Figures 10A-B). In the DSS model, the same cytokines showed a very similar behaviour (Figures 10C-D) and the PDRNs decreased the expression thereof.

The results from the experimental tests allow for predicting the use of PDRN compositions for the therapeutic treatment of inflammatory bowel disease, particularly Crohn's disease and ulcerative colitis, in a patient suffering from these diseases by administering an effective amount of said composition.

In particular, the therapeutic treatment comprises the administration of polynucleotide or PDRN compositions by the intramuscular route. For this purpose, previously described PDRN compositions which are commercially available under the trade name Placentex@ intact can be used, for example by daily administration of from 1 to 3 vials of the commercial product.

Other administration forms of the drug may comprise rectal foam or gel formulations, as well as oral administrations, preferably in the form of gastro-resistant tablets. For these administration forms, pharmaceutically acceptable carriers that are commercially available can be used in association with the active ingredient.

## Claims

1. A composition comprising a mixture of polydeoxyribonucleotides (PDRNs) extracted from fish sperm or plants, for use in the therapeutic treatment of inflammatory bowel disease in a human subject, wherein said PDRN mixture is devoid of informational capacity as a result of prior treatment by at least partial depurination and is a mixture of PDRNs polymer chains, with different molecular weights with chain lengths of from 50 to 2000 base pairs and comprises a mixture of polymer chains having a distribution of molecular weights between 0.5 kD and 3000 kD.

2. A composition for use according to claim 1 for the therapeutic treatment of Crohn's disease or ulcerative colitis, by parenteral, oral or rectal administration.

3. A composition for use according to claim 1 or 2, wherein said polydeoxyribonucleotides are depurinised in a percentage comprised between 1 and 5% of purine bases removed, based on the total of the purine bases originally present.

4. A composition for use according to any of claims 1 to 3, wherein said mixture of polydeoxyribonucleotides has a number average molecular weight of from 250 to 400 kD.

5. A composition for use according to any one of the preceding claims, comprising a pharmaceutically acceptable carrier.

6. A composition for use according to any one of the preceding claims, in the form of a rectal foam or gel.

7. A composition for use according to any one of claims 1 to 5 in the form of a coated tablet for oral administration.

8. A composition for use according to any one of claims 1 to 5 in the form of a solution for injection comprising PDRNs in an aqueous saline solution.

9. A composition for use according to any one of claims 1 to 8, wherein said composition is administered in an amount effective to reduce the symptoms of said inflammatory bowel disease.

## Patentansprüche

1. Zusammensetzung, aufweisend eine Mischung aus Polydesoxyribonukleotiden (PDRN), die aus Fischspermien oder Pflanzen extrahiert sind, zur Verwendung in der therapeutischen Behandlung einer entzündlichen Darmerkrankung bei einem Menschen, wobei die PDRN-Mischung ohne Informationsgehalt ist, infolge einer vorherigen Behandlung durch eine zumindest teilweise Depurinierung, und eine Mischung aus PDRN-Polymerketten mit unterschiedlichen Molekulargewichten mit Kettenlängen von 50 bis 2000 Basenpaaren ist und eine Mischung von Polymerketten mit einer Verteilung der Molekulargewichte zwischen 0,5 kD und 3000 kD aufweist.

2. Zusammensetzung zur Verwendung nach Anspruch 1 zur therapeutischen Behandlung von Morbus Crohn oder Colitis ulzerosa durch parenterale, orale oder rektale Verabreichung.

3. Zusammensetzung zur Verwendung nach Anspruch 1 oder 2, wobei die Polydesoxyribonukleotide zu einem Prozentsatz depuriniert sind, der zwischen 1 und 5 % der entfernten Purinbasen umfasst, basierend auf der Gesamtheit der ursprünglich vorhandenen Purinbasen.

4. Zusammensetzung zur Verwendung nach einem der Ansprüche 1 bis 3, wobei die Mischung aus Polydesoxyribonukleotiden ein zahlenmittleres Molekulargewicht von 250 bis 400 kD hat.

5. Zusammensetzung zur Verwendung nach einem der vorangehenden Ansprüche, aufweisend ein pharmazeutisch vertretbares Trägermaterial.

6. Zusammensetzung zur Verwendung nach einem der vorangehenden Ansprüche in Form eines Rektalschaums oder -gels.

7. Zusammensetzung zur Verwendung nach einem der Ansprüche 1 bis 5 in Form einer Filmtablette zur oralen Verabreichung.

8. Zusammensetzung zur Verwendung nach einem der Ansprüche 1 bis 5 in Form einer PDRN in einer wässrigen Kochsalzlösung aufweisenden Lösung zur Injektion.

9. Zusammensetzung zur Verwendung nach einem der Ansprüche 1 bis 8, wobei die Zusammensetzung in einer Menge verabreicht wird, die zur Verringerung der Symptome der entzündlichen Darmerkrankung wirksam ist.

## Revendications

1. Composition comprenant un mélange de polydésoxyribonucléotides (PDRN) extraits de sperme de poisson ou de plantes, pour une utilisation dans le traitement thérapeutique d'une maladie inflammatoire de l'intestin chez un sujet humain, dans laquelle ledit mélange de PDRN est dépourvu de capacité informationnelle suite à un traitement préalable par au moins une dépurination partielle et est un mélange de chaînes polymères de PDRN, ayant différents poids moléculaires avec des longueurs de chaînes de 50 à 2 000 paires de base et comprend un mélange de chaînes polymères ayant une répartition de poids moléculaires comprise entre 0,5 kD et 3 000 kD.

2. Composition pour une utilisation selon la revendication 1 pour le traitement thérapeutique de la maladie de Crohn ou de la rectocolite hémorragique, par administration parentérale, orale ou rectale.

3. Composition pour une utilisation selon la revendication 1 ou 2, dans laquelle lesdits polydésoxyribonucléotides sont dépurinés en un pourcentage compris entre 1 et 5 % des bases puriques éliminées, sur la base du total des bases puriques initialement présentes.

4. Composition pour une utilisation selon l'une quelconque des revendications 1 à 3, dans laquelle ledit mélange de polydésoxyribonucléotides a un poids moléculaire moyen en nombre de 250 à 400 kD.

5. Composition pour une utilisation selon l'une quelconque des revendications précédentes, comprenant un véhicule pharmaceutiquement acceptable.

6. Composition pour une utilisation selon l'une quelconque des revendications précédentes, sous la forme d'une mousse ou d'un gel rectal.

7. Composition pour une utilisation selon l'une quelconque des revendications 1 à 5 sous la forme d'un comprimé enrobé pour administration orale.

8. Composition pour une utilisation selon l'une quelconque des revendications 1 à 5 sous la forme d'une solution pour injection comprenant des PDRN dans une solution saline aqueuse.

9. Composition pour une utilisation selon l'une quelconque des revendications 1 à 8, dans laquelle ladite composition est administrée en une quantité efficace pour réduire les symptômes de ladite maladie inflammatoire de l'intestin.
